# EUROPEAN PATENT SPECIFICATION

(11) **EP 1 697 874 B1**
(45) Date of publication and mention of the grant of the patent: **23.11.2011**
(21) Application number: 04708200.3
(22) Date of filing: 04.02.2004
(51) Int. Cl.: G06G 7/48, G06G 7/60, A61B 5/05, A61B 5/103, A61B 5/117, A61F 2/08

(54) **Computer-assisted knee replacement apparatus**
Computerunterstützte Knieersatzvorrichtung
Appareil de remplacement d'un genou assisté par ordinateur

(30) Priority: 04.02.2003 US 444988 P
(43) Date of publication of application: 06.09.2006
(73) Proprietor: Z-Kat, Inc., Hollywood, FL 33020 (US)
(72) Inventor: MARQUART, Joel, Pembroke Pines, FL 33028 (US); ILLSLEY, Scott, Waterloo, Ontario N2L 2J2 (CA); ARATA, Louis K., Mentor, OH 44060 (US); HAND, Randall, Clinton, MS 39056 (US); QUAID III, Arthur E., Ft. Lauderdale, FL 33312 (US); ABOVITZ, Rony A., Hollywood, FL 33021 (US); GEROVICH, Oleg E., Cedar Knolls, NJ 07927 (US); MCKALE, James M., Leesburg, IN 46538 (US)
(74) Representative: Kenrick, Mark Lloyd
(86) International application number: PCT/US2004/003130
(87) International publication number: WO 2004/070580

(56) References cited:
- EP-A1- 1 226 788
- WO-A2-02/067783
- DE-A1- 10 031 887
- US-A- 4 433 961
- US-A- 5 682 886
- US-A- 5 769 092
- US-A- 5 769 640
- US-A- 5 880 976
- US-A1- 2002 038 085
- US-A1- 2002 107 522
- US-A1- 2004 030 245
- US-B1- 6 205 411
- US-B1- 6 228 089
- US-B1- 6 385 475
- US-B1- 6 385 475
- US-B1- 6 450 978
- US-B1- 6 533 737
- US-B1- 6 701 174
- US-B1- 6 711 432

## Description

### TECHNICAL FIELD OF THE INVENTION

The present invention relates generally to the field of computer-assisted medical systems and, more particularly, to a computer-assisted knee replacement apparatus .

### BACKGROUND OF THE INVENTION

Image-based surgical navigation systems display the positions of surgical tools with respect to preoperative (prior to surgery) or intraoperative (during surgery) image datasets. Two and three dimensional image data sets are used, as well as time-variant images data (i.e. multiple data sets take at different times). Types of data sets that are primarily used include two-dimensional fluoroscopic images and three-dimensional data sets include magnetic resonance imaging (MRI) scans, computer tomography (CT) scans, positron emission tomography (PET) scans, and angiographic data. Intraoperative images are typically fluoroscopic, as a C-arm fluoroscope is relatively easily positioned with respect to patient and does not require that a patient be moved. Other types of imaging modalities require extensive patient movement and thus are typically used only for preoperative and post-operative imaging.

The most popular navigation systems make use of a tracking or localizing system to track tools, instruments and patients during surgery. These systems locate in predefined coordinate space specially recognizable markers or elements that are attached or affixed to, or possibly inherently a part of, an object such as an instrument or a patient. The elements can take several forms, including those that can be located using optical (or visual), magnetic, or acoustical methods. Furthermore, at least in the case of optical or visual systems, the location of an object's position may be based on intrinsic features or landmarks that, in effect, function as recognizable elements. The elements will have a known, geometrical arrangement with respect to, typically, an end point and/or axis of the instrument. Thus, objects can be recognized at least in part from the geometry of the elements (assuming that the geometry is unique), and the orientation of the axis and location of endpoint within a frame of reference deduced from the positions of the elements.

A typical optical tracking system functions primarily in the infrared range. They usually include a stationary stereo camera pair that is focused around the area of interest and sensitive to infrared radiation. Elements emit infrared radiation, either actively or passively. An example of an active element is a light emitting diode (LED). An example of a passive element is a reflective element, such as ball-shaped element with a surface that reflects incident infrared radiation. Passive systems require an infrared radiation source to illuminate the area of focus. A magnetic system may have a stationary field generator that emits a magnetic field that is sensed by small coils integrated into the tracked tools.

Most computer-assisted surgery (CAS) systems are capable of continuously tracking, in effect, the position of tools (sometimes also called instruments). With knowledge of the position of the relationship between the tool and the patient and the patient and an image data sets, a system is able to continually superimpose a representation of the tool on the image in the same relationship to the anatomy in the image as the relationship of the actual tool to the patient's anatomy. To obtain these relationships, the coordinate system of the image data set must be registered to the relevant anatomy of the actual patient and portions of the of the patient's anatomy in the coordinate system of the tracking system. There are several known registration methods. A general purpose computer-assisted surgery system providing tool tracking is disclosed in US Patent No. US6,947,783, on which the preamble of claim 1 is based,

In CAS systems that are capable of using two-dimensional image data sets, multiple images are usually taken from different angles and registered to each other so that a representation of the tool or other object (which can be real or virtual) can be, in effect, projected into each image. As the position of the object changes in three-dimensional space, its projection into each image is simultaneously updated. In order to register two or more two-dimensional data images together, the images are acquired with what is called a registration phantom in the field of view of the image device. In the case of a two-dimensional fluoroscopic images, the phantom is a radio-translucent body holding radio- opaque fiducials having a known geometric relationship. Knowing the actual position of the fiducials in three-dimensional space when each of the images are taken permits determination of a relationship between the position of the fiducials and their respective shadows in each of the images. This relationship can then be used to create a transform for mapping between points in three-dimensional space and each of the images. By knowing the positions of the fiducials with respect to the tracking system's frame of reference, the relative positions of tracked tools with respect to the patient's anatomy can be accurately indicated in each of the images, presuming the patient does not move after the image is acquired, or that the relevant portions of the patient's anatomy are tracked. A more detailed explanation of registration of fluoroscopic images and coordination of representations of objects in patient space superimposed in the images is found in United States Patent 6,198,794 of Peshkin, et al., entitled "Apparatus and method for planning a stereotactic surgical procedure using coordinated fluoroscopy."

### SUMMARY OF THE INVENTION

The invention is generally directed to improved computer-implemented apparatus for further reducing the invasiveness of surgical procedures, eliminating or reducing the need for external fixtures in certain surgical procedures, and/or improving the precision and/or consistency of surgical procedures. The invention finds particular advantage in orthopedic procedures involving implantation of devices, though it may also be used in connection with other types of surgical procedures.

The computer-assisted knee replacement apparatus of the present invention assists with performing a total knee replacement procedure. In this embodiment, the knee replacement application provides implant sizing corresponding to the subject. The knee replacement application also provides planning and guiding of femoral and/or tibial resection preparation.

### BRIEF DESCRIPTION OF THE DRAWINGS

For a more complete understanding of the present invention and the advantages thereof, reference is now made to the following descriptions taken in connection with the accompanying drawings in which:
FIGURE 1 is a block diagram illustrating an exemplary computer-assisted surgery system;
FIGURE 2 is a flow chart of basic steps of an application program for assisting with or guiding the planning of, and navigation during, a total knee replacement procedure; and
FIGURES 3-17 are representative screen images of graphical user interface pages generated and displayed by the application program of FIGURE 2.

### DETAILED DESCRIPTION OF THE DRAWINGS

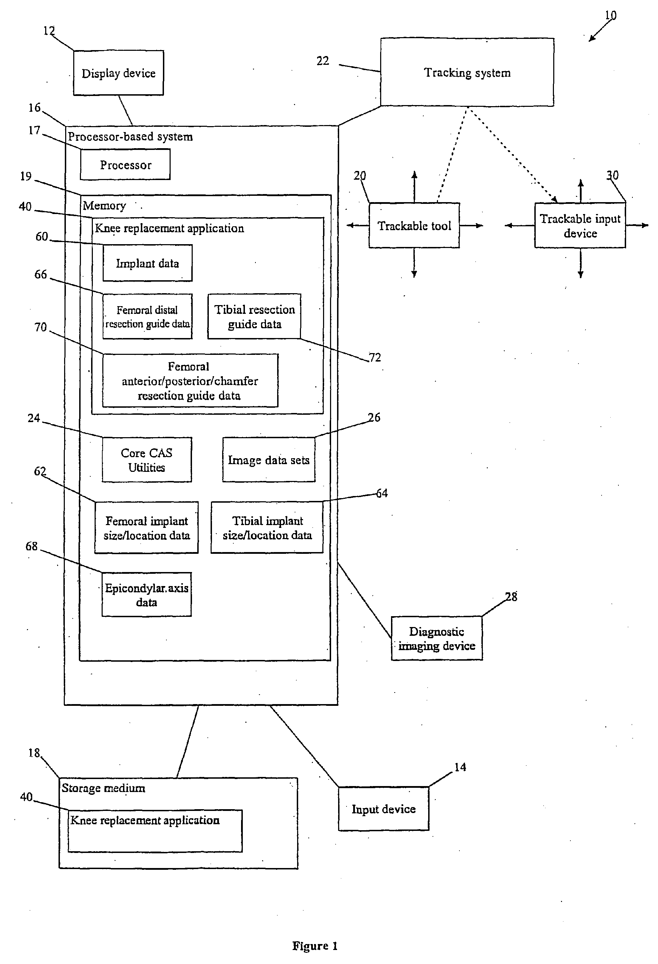

The preferred embodiments of the present invention and the advantages thereof are best understood by referring to FIGURES 1-17 of the drawings, like numerals being used for like and corresponding parts of the various drawings.

FIGURE 1 is a block diagram of an exemplary computer-assisted surgery (CAS) system 10. CAS system 10 comprises a display device 12, an input device 14, and a processor-based system 16, for example a computer. Display device 12 may be any display device now known or later developed for displaying two-dimensional and/or three-dimensional diagnostic images, for example, a monitor, a touch screen, a wearable display, a projection display, a head-mounted display, stereoscopic views, a holographic display, a display device capable of displaying image(s) projected from an image projecting device, for example a projector, and/or the like. Input device 14 may be any input device now known or later developed, for example, a keyboard, a mouse, a trackball, a trackable probe, and/or the like. The processor-based system 16 is preferably programmable and includes one or more processors 17, working memory 19 for temporary program and data storage that will be used primarily by the processor, and storage for programs and data, preferably persistent, such as a disk drive. Removable media storage medium 18 can also be used to store programs and/or data transferred to or from the processor-based system 16. The storage medium 18 may include a floppy disk, an optical disc, or any other type of storage medium now known or later developed.

Tracking system 22 continuously determines, or tracks, the position of one or more trackable elements disposed on, incorporated into, or inherently a part of surgical instruments or tools 20 with respect to a three-dimensional coordinate frame of reference. With information from the tracking system 22 on the location of the trackable elements, CAS system 10 is programmed to be able to determine the three-dimensional coordinates of an endpoint or tip of a tool 20 and, optionally, its primary axis using predefined or known (e.g. from calibration) geometrical relationships between trackable elements on the tool and the endpoint and/or axis of the tool 20. A patient, or portions of the patient's anatomy, can also be tracked by attachment of arrays of trackable elements.

The CAS system 10 can be used for both planning surgical procedures (including planning during surgery) and for navigation. It is therefore preferably programmed with software for providing basic image guided surgery functions, including those necessary for determining the position of the tip and axis of instruments and for registering a patient and preoperative and/or intraoperative diagnostic image data sets to the coordinate system of the tracking system. The programmed instructions for these functions are indicated as core CAS utilities 24. These capabilities allow the relationship of a tracked instrument to a patient to be displayed and constantly updated in real time by the CAS system 10 overlaying a representation of the tracked instrument on one or more graphical images of the patient's anatomy on display device 12. The graphical images may be a virtual representation of the patient's anatomy or may be constructed from one or more stored image data sets 26 acquired from a diagnostic imaging device 28. The imaging device may be a fluoroscope, such as a C-arm fluoroscope, capable of being positioned around a patient laying on an operating table. It may also be a MR, CT or other type of imaging device in the room or permanently located elsewhere. Where more than one image is shown, as when multiple fluoroscopic images are simultaneously displayed of display device 12, the representation of the tracked instrument or tool is coordinated between the different images. However, CAS system 10 can be used in some procedures without the diagnostic image data sets, with only the patient being registered. Thus, the CAS system 10 may need not to support the use diagnostic images in some applications - i.e., an imageless application.

Furthermore, as disclosed herein, the CAS system 10 may be used to run application-specific programs that are directed to assisting a surgeon with planning and/or navigation during specific types of procedures. For example, the application programs may display predefined pages or images corresponding to specific steps or stages of a surgical procedure. At a particular stage or part of a program, a surgeon may be automatically prompted to perform certain tasks or to define or enter specific data that will permit, for example, the program to determine and display appropriate placement and alignment of instrumentation or implants or provide feedback to the surgeon. Other pages may be set up to display diagnostic images for navigation and to provide certain data that is calculated by the system for feedback to the surgeon. Instead of or in addition to using visual means, the CAS system 10 could also communicate information in ways, including using audibly (e.g. using voice synthesis) and tactilely, such as by using a haptic interface type of device. For example, in addition to indicating visually a trajectory for a drill or saw on the screen, the CAS system 10 may feedback to a surgeon information whether he is nearing some object or is on course with a audible sound or by application of a force or other tactile sensation to the surgeon's hand.

To further reduce the burden on the surgeon, the program may automatically detect the stage of the procedure by recognizing the instrument picked up by a surgeon and move immediately to the part of the program in which that tool is used. Application data generated or used by the application may also be stored in processor-based system 16.

Various types of user input methods can be used to improve ease of use of the CAS system 10 during surgery. One example is the use the use of speech recognition to permit a doctor to speak a command. Another example is the use of a tracked object to sense a gesture by a surgeon, which is interpreted as an input to the CAS system 10. The meaning of the gesture could further depend on the state of the CAS system 10 or the current step in an application process executing on the CAS system 10. Again, as an example, a gesture may instruct the CAS system 10 to capture the current position of the object. One way of detecting a gesture is to occlude temporarily one or more of the trackable elements on the tracked object (e.g. a probe) for a period of time, causing loss of the CAS system's 10 ability to track the object. A temporary visual occlusion of a certain length (or within a certain range of time), coupled with the tracked object being in the same position before the occlusion and after the occlusion, would be interpreted as an input gesture. A visual or audible indicator that a gesture has been recognized could be used to provide feedback to the surgeon.

Yet another example of such an input method is the use of tracking system 22 in combination with one or more trackable data input devices 30. Defined with respect to the trackable input device 30 are one or more defined input areas, which can be two-dimensional or three-dimensional. These defined input areas are visually indicated on the trackable input device 30 so that a surgeon can see them. For example, the input areas may be visually defined on an object by representations of buttons, numbers, letters, words, slides and/or other conventional input devices. The geometric relationship between each defined input area and the trackable input device 30 is known and stored in processor-based system 16 Thus, the processor 17 can determine when another trackable object touches or is in close proximity a defmed input area and recognize it as an indication of a user input to the processor based system 16. For example, when a tip of a tracked pointer is brought into close proximity to one of the defined input areas, the processor-based system 16 will recognize the tool near the defined input area and treat it as a user input associated with that defined input area. Preferably, representations on the trackable user input correspond user input selections (e.g. buttons) on a graphical user interface on display device 12. The trackable input device 30 may be formed on the surface of any type of trackable device, including devices used for other purposes. In a preferred embodiment, representations of user input functions for graphical user interface are visually defined on a rear, flat surface of a base of a tool calibrator.

Processor-based system 16 is, in one example, a programmable computer that is programmed to execute only when single-use or multiple-use software is loaded from, for example, removable media 18. The software would include, for example the application program for use with a specific type of procedure. The application program can be sold bundled with disposable instruments specifically intended for the procedure. The application program would be loaded into the processor-based system 16 and stored there for use during one (or a defined number) of procedures before being disabled. Thus, the application program need not be distributed with the CAS system 10. Furthermore, application programs can be designed to work with specific tools and implants and distributed with those tools and implants. Preferably, also, the most current core CAS utilities 24 may also be stored with the application program. If the core CAS utilities 24 on the processor-based system 16 are outdated, they can be replaced with the most current utilities.

In FIGURE 1, the application program comprises a total knee replacement application 40 for assisting with, planning, and guiding a total knee replacement procedure. The knee replacement application 40 provides a series of displayable images and corresponding instructions or guidelines for performing the knee replacement procedure. The knee replacement application 40 may be loaded into the processor-based system 16 from the media storage device 18. Processor-based system 16 may then execute the knee replacement application 40 solely from memory 19 or portions of the application 40 may be accessed and executed from both memory 19 and the storage medium 18.

Briefly, in one embodiment, the knee replacement application 40 cooperates with a tracking system 22 to plan femoral and/or tibial implant sizes for the subject. The knee replacement application 40 also cooperates with tracking system 22 to assist with planning, selecting and preparing femoral and/or tibial resections by locating and positioning cutting guides and other tools relative to the subject's knee to minimize the invasiveness of the procedure and increase the accuracy of knee implant placement. Application 40 also enables a user to review kinematic parameters of the subject's knee.

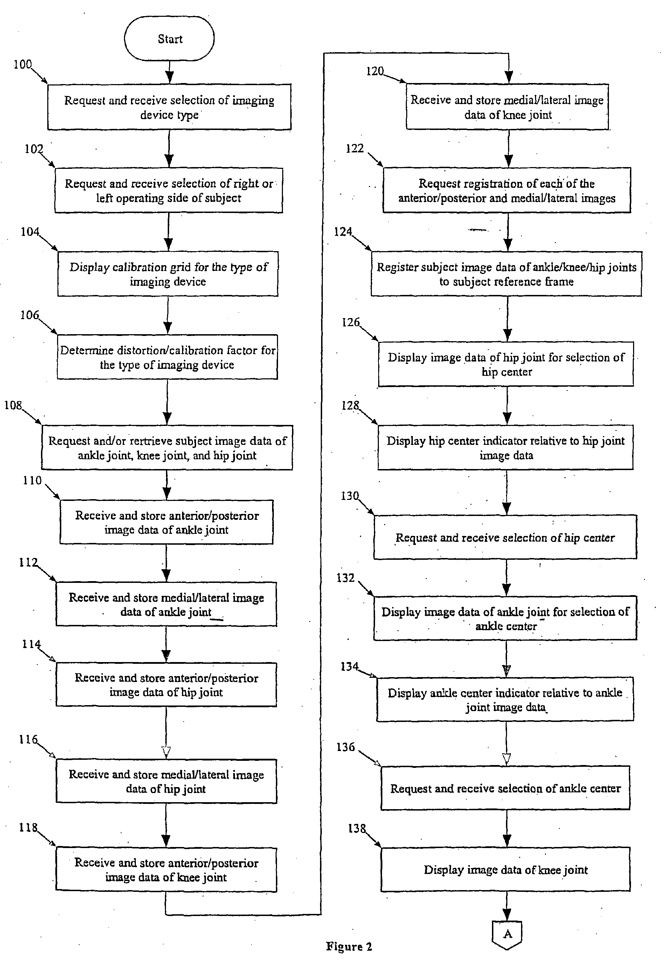

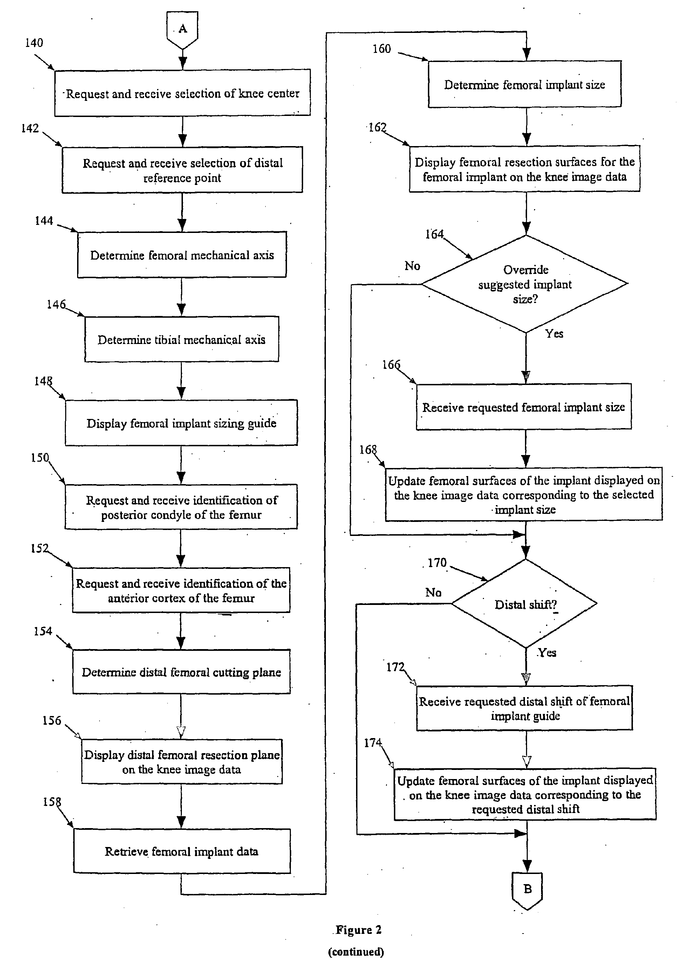

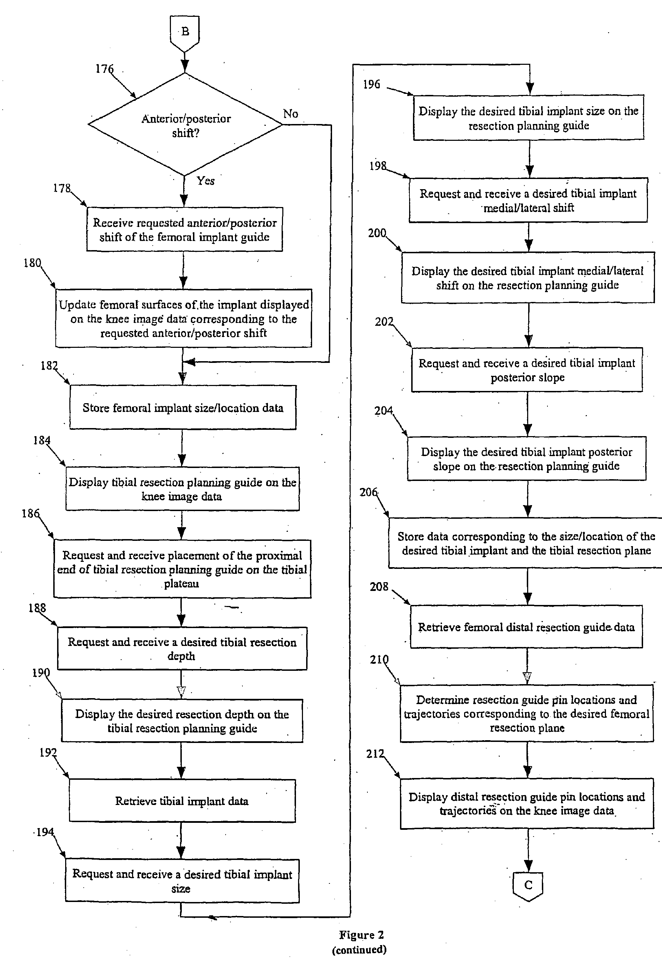

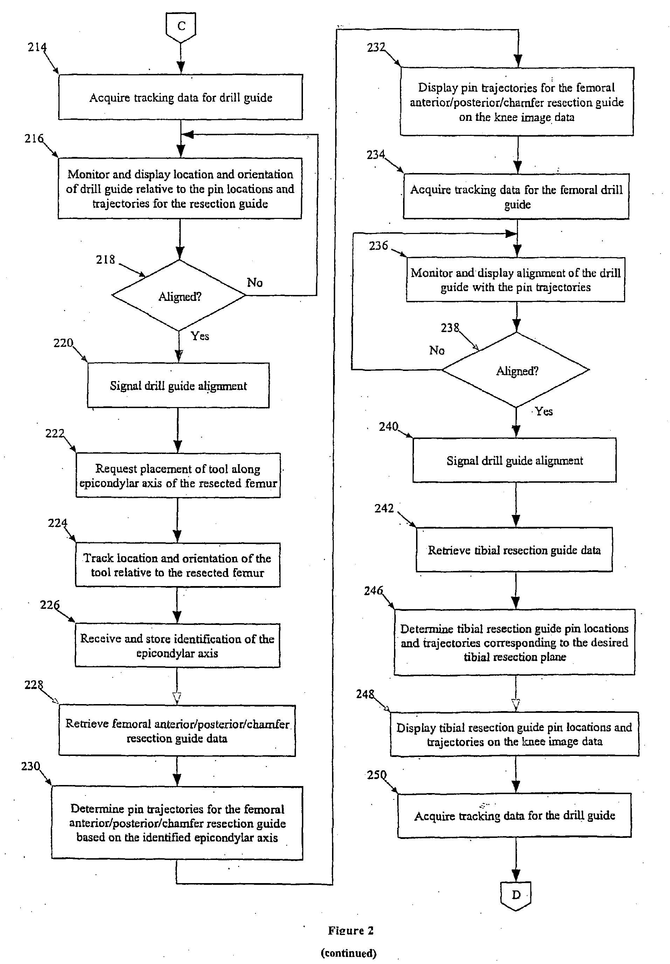

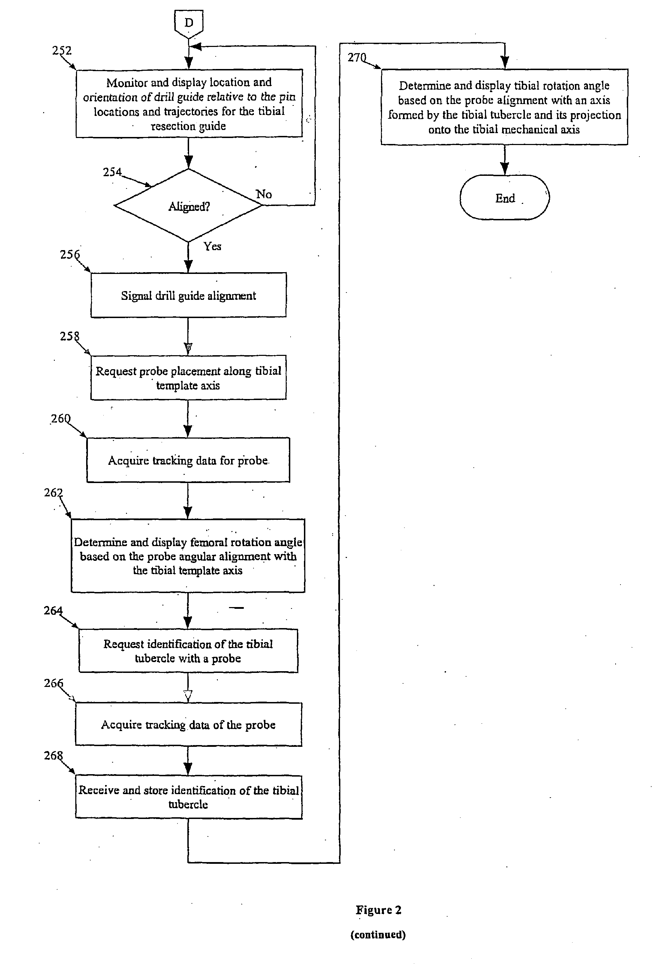

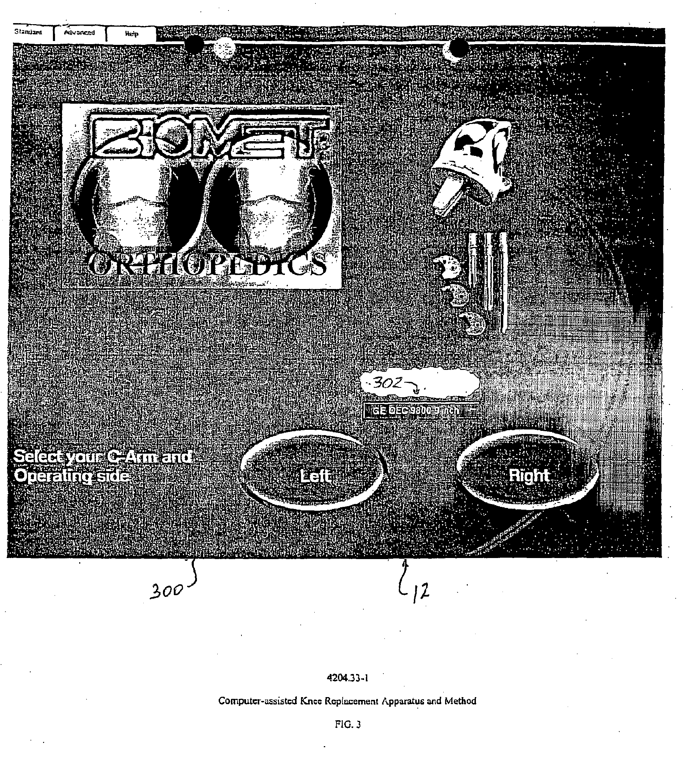

FIGURE 2 is a flowchart illustrating an exemplary embodiment of a series of steps of the knee replacement application 40 in accordance with the present invention. The method begins at step 100, where knee replacement application 40 displays a procedure initiation screen 300, as best illustrated in FIGURE 3, and requests the user to select a type of imaging device 28 or type of images to be used for the procedure. For example, as illustrated in FIGURE 3, the knee replacement application 40 displays a menu or listing, indicated generally by 302, of various C-arm fluoroscopes or other types of intraoperative imaging devices 28 that may used for the total knee replacement procedure. Though fluoroscopic is presently the preferred imaging mode for creating intraoperative images, other types of imaging devices 28 can also be used. Furthermore, preoperative diagnostic images may be used in some circumstances including two-dimensional and/or three-dimensional image data sets 26. The image data sets 36 may also comprise a time component or dimension such that changes in the physical structure of the subject may be displayed over time. Additionally, as described above, the application need not support the use diagnostic images in some applications. For example, the knee replacement application may be configured to illustrate a virtual representation of the subject's knee on the display device 12 such that knee implant sizing and location, as well as knee preparation procedures for the implants, may be performed using the virtual representation, thereby providing a subject-imageless guidance, planning and assistance application. In this case, the user may select or identify various locations on the subject's knee with a trackable tool 20 such that the tracking system 22 may register and identify the selected locations on the displayed virtual representation. The displayed virtual representation of the subject's knee may then be used to perform knee implant sizing and locating, as well as planning and guiding required femoral and/or tibial preparation procedures for the implants. As will be described in more detail below, in the illustrated embodiment, fluoroscopic images are acquired of the subject and used by knee replacement application 40 to guide various procedures of the knee replacement procedure as well as sizing and locating the knee replacement implants. The knee replacement application 40 provides output to the user, such as requests or instructions, in the form of audible signals or visual signals, such as via display device 12. The knee replacement application 40 may also provide haptic output to the user during the procedure. For example, as will be described in greater detail below, application 40 provides the user with feedback corresponding to alignment information of a trackable tool 20 or implant guide relative to the subject. The knee replacement application 40 may be configured to provide haptic feedback to the user during these and other procedural phases of the knee replacement procedure. After the knee replacement application 40 receives a selection of the type of imaging device 28, the method proceeds to step 102, where the knee replacement application 40 requests the selection of either the right or left operating side of the subject. After receiving a selection of the right or left operating side of the subject by the user, the method proceeds to step 104.

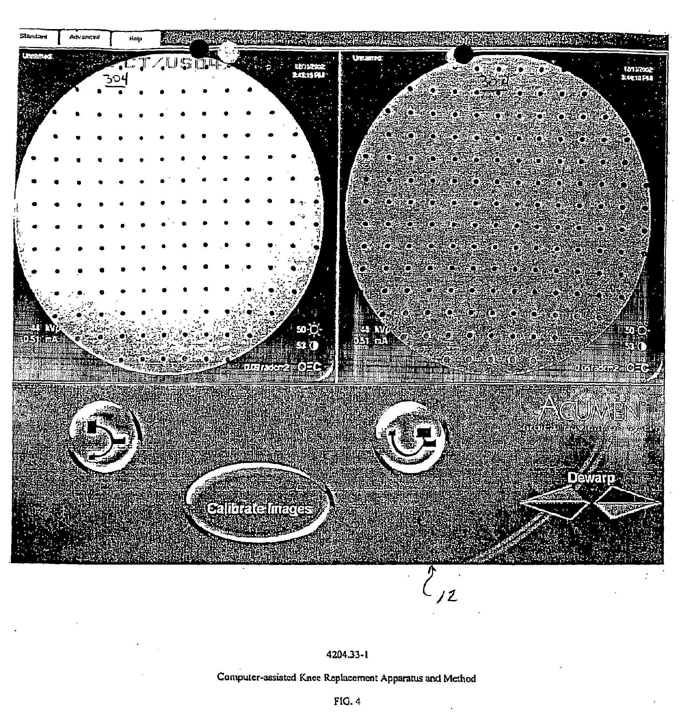

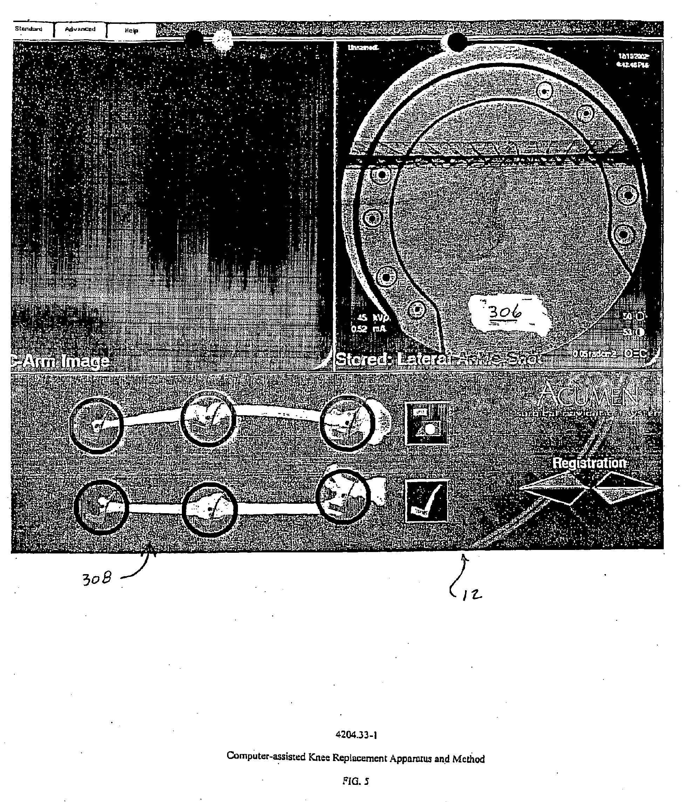

At step 104, the knee replacement application 40 displays a calibration grid 304, as best illustrated in FIGURE 4. As is well known in the art, a calibration grid is used to determine the distortion inherent in images from the actual imaging device selected for use. It is well known, for example, that fluoroscopic images are inherently distorted or warped and must be dewarped. At step 106, a distortion or calibration factor for the selected type of imaging device 28 is determined and applied. At step 108, the knee replacement application 40 requests or retrieves subject image data 26 for the ankle joint, the knee joint, and the hip joint of the subject, as best illustrated in FIGURE 5. As illustrated in FIGURE 5, the left portion of the image displayed on display device 12 comprises a real-time image of the subject using a selected type of fluoroscopic imaging device 28. The user may then use an input device 14, a touch screen associated with display device 12, or another method of inputting information into processor-based system 16 to select or capture a desired real-time image, which is then stored and displayed in the right portion of the image illustrated in FIGURE 5, indicated generally by 306. As illustrated in FIGURE 5, the knee replacement application 40 requests anterior/posterior image data and medial/lateral image data for the ankle joint, knee joint and hip joint. Thus, at step 110, the knee replacement application 40 receives and stores anterior/posterior image data of the ankle joint. At step 112, the knee replacement application 40 receives and stores medial/lateral image data of the ankle joint. At step 114, the knee replacement application 40 receives and stores anterior/posterior image data of the hip joint. At step 116, the knee replacement application 40 receives and stores medial/lateral image data of the hip joint. At step 118, the knee replacement application 40 receives and stores anterior/posterior image data of the knee joint. At step 120, the knee replacement application 40 receives and stores medial/lateral image data of the knee joint.

At step 122, the knee replacement application 40 requests registration of each of the anterior/posterior and medial/lateral images of the knee, ankle, and hip joints relative to the subject reference frame. For example, trackable element arrays may be secured or otherwise coupled to the subject, such as secured to the femur and tibia of the subject, such that tracking system 22 may register each image to the subject reference frame. Thus, at step 124, tracking system 22 registers the image data 26 of the ankle, knee, and hip joints to the subject reference frame. As illustrated in FIGURE 5, the knee replacement application 40 indicates on display device 12 the status of image data acquisition and registration for the anterior/posterior and medial/lateral images of each of the ankle, knee, and hip joint by indicating a check mark upon completion of image data acquisition and registration, indicated generally by 308. The image data 26 of the ankle, knee and hip joint are also correlated to provide real-time three-dimensional tracking of tools 20 or other devices relative to the subject.

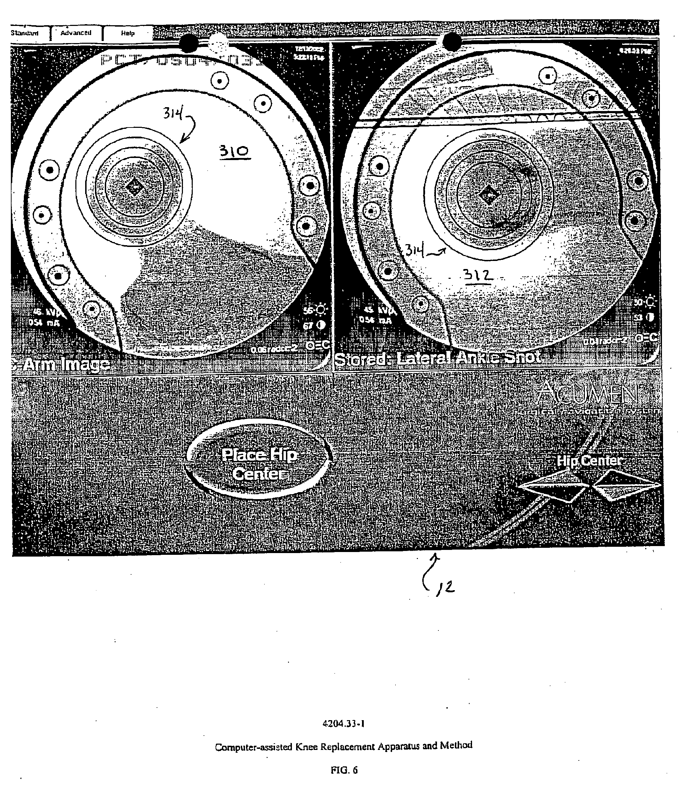

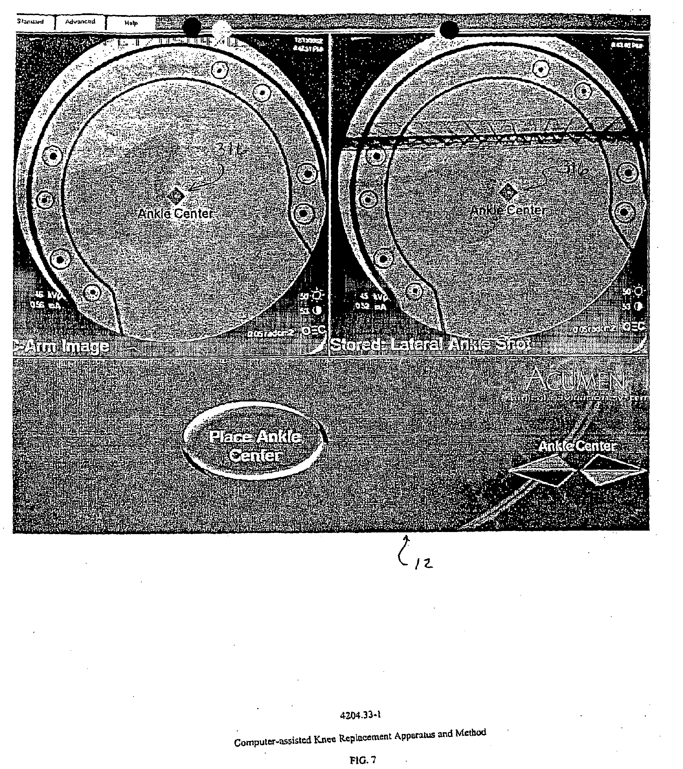

At step 126, the knee replacement application 40 displays on display device 12 anterior/posterior image data 26, as shown in the left portion of FIGURE 6 and indicated generally by 310, and medial/lateral image data 26, as shown in the right portion of FIGURE 6 and indicated generally by 312, of the hip joint of the subject. For ease of description, fluoroscopic images illustrated in the following FIGURES 7-15 shall represent anterior/posterior and medial/lateral views positioned in the corresponding portions of the displayed FIGURES 7-15 as described above. At step 128, the knee replacement application 40 displays a hip center indicator 314 relative to the hip joint image data 26 such that a user may manipulate the hip center indicator 314 relative to the displayed hip image data 26 to locate and select a center of the femoral hip socket bone structure. At step 130, the knee replacement application 40 requests and receives a selection of the hip center in response to the user manipulating the hip center indicator 314 to a desired position and entering the selection with an input device 14, a touch screen associated with display device 12, or other method of selection.

At step 132, the knee replacement application 40 displays image data 26 of the ankle joint of the subject for selection of the ankle center, as best illustrated in FIGURE 7. At step 134, the knee replacement application 40 displays an ankle center indicator 316 relative to the fluoroscopic ankle joint image data 26 displayed on display device 12 such that a user may manipulate the ankle center indicator 316 relative to the displayed ankle joint image data 26 to select the ankle center. At step 136, the knee replacement application 40 requests and receives a selection of the ankle center.

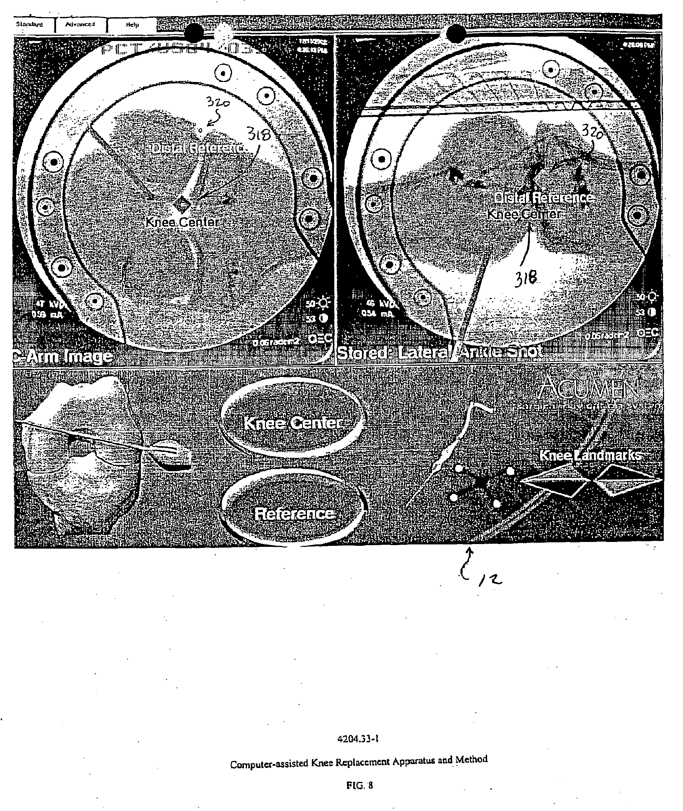

At step 138, the knee replacement application 40 displays the image data 26 of the knee joint on display device 12. At step 140, the knee replacement application 40 requests and receives a selection of the knee center. For example, as best illustrated in FIGURE 8, the knee replacement application 40 may display a knee center indicator 318 relative to the knee image data 26 such that a user may manipulate the knee center indicator 318 relative to the knee image data 26 of the knee joint to identify and locate the knee center. However, it should also be understood that a trackable tool 20 may also be used to locate and identify the knee center. Additionally, the knee center may also be defined by or as different points relative to the femur and/or tibia by the user. At step 142, the knee replacement application 40 also requests and receives selection of a distal preference point, such as the most distal point of the condyle of the femur. As best illustrated in FIGURE 8, the knee replacement application 40 may display a distal reference indicator 320 relative to the knee image data 26 such that a user may manipulate the distal reference indicator 320 relative to the knee joint image data 26 to identify and select the distal reference point. However, it should be understood that a trackable tool 20 may also be used by the user to identify and select the distal reference point. At step 144, the knee replacement application 40 determines the femoral mechanical axis using the hip center data and the knee center data. At step 146, the knee replacement application 40 determines the tibial mechanical axis using the ankle center data and the knee center data.

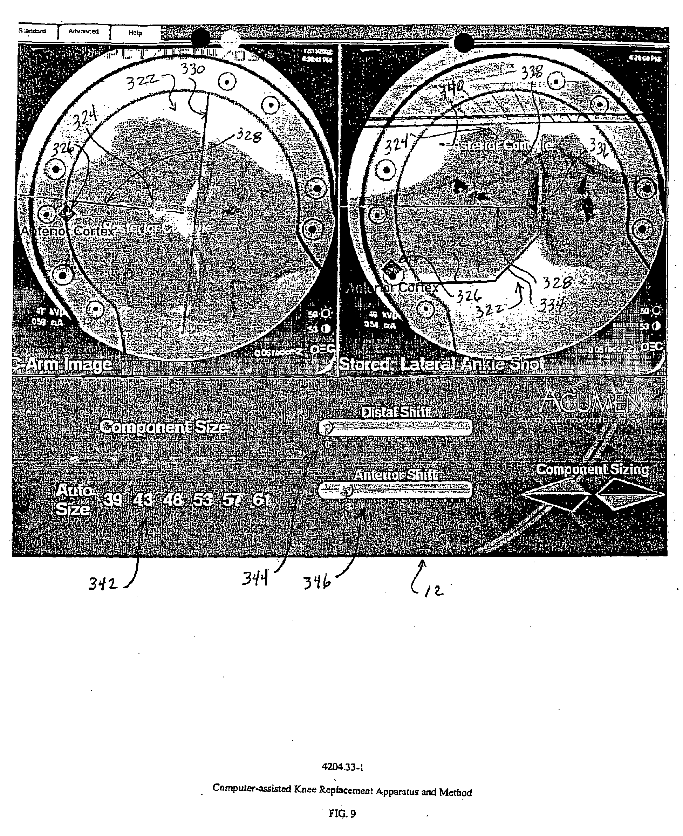

At step 148, the knee replacement application 40 displays a femoral implant sizing guide 322 on display device 12 relative to the knee image data 26, as best illustrated in FIGURE 9. At step 150, the knee replacement application 40 requests and receives identification and selection of the posterior condyle of the femur. As illustrated in FIGURE 9, the knee replacement application 40 may display a posterior condyle indicator 324 relative to the displayed knee image data 26 such that a user may manipulate the posterior condyle indicator 324 relative to the knee image data 26 to identify and select the posterior condyle. However, it should be understood that a trackable tool 20 may also be used to identify and select the posterior condyle. At step 152, the knee replacement application 40 requests and receives identification and selection of the anterior cortex of the femur. As best illustrated in FIGURE 9, the knee replacement application 40 displays an anterior cortex indicator 326 relative to the displayed knee image data 26 such that the user may manipulate the anterior cortex indicator 326 relative to the knee image data to identify and select the anterior cortex of the subject knee.

At step 154, the knee replacement application 40 automatically determines the distal femoral resection plane based on the determined femoral mechanical axis and the location of the posterior condyle and anterior cortex. At step 156, the knee replacement application 40 displays the distal femoral resection plane on the displayed knee image data 26. For example, referring to FIGURE 9, the line indicated by 328 illustrated in the anterior/posterior and the medial/lateral views represents the femoral mechanical axis, and the line indicated by 330 illustrated in the anterior/posterior image view represents the distal femoral resection plane.

At step 158, the knee replacement application 40 retrieves implant data 60 corresponding to available femoral implants. For example, the femoral implant data 60 may comprise information associated with various sizes of femoral implants such that the geometric characteristics of the various femoral implants may be displayed relative to the displayed knee image data 26. At step 160, the knee replacement application 40 automatically determines a suggested femoral implant size corresponding to the determined distal femoral resection plane and the locations of the posterior condyle and anterior cortex. At step 162, the knee replacement application 40 displays the femoral resection surfaces for the femoral implant on the relative to the displayed knee image data 26. For example, referring to FIGURE 9, the knee replacement application displays a femoral implant sizing guide 322 as illustrated by the series of lines indicated generally by 332, 334, 336, 338 and 340 shown in the medial/lateral view of FIGURE 9, of which the distal femoral resection plane is also shown in the anterior/posterior view of FIGURE 9, which represent the target femoral resection surfaces for the femur corresponding to a particular femoral implant. At decisional step 164, a determination is made whether the user desires to override the suggested femoral implant size. If the user does not desire to override the suggested implant size, the method proceeds from step 164 to decisional step 170. If the user desires to override the suggested femoral implant size, the method proceeds from step 164 to step 166, where the knee replacement application 40 receives a requested or desired femoral implant size. For example, as best illustrated in FIGURE 9, the knee replacement application 40 may display various sizing options to the user, indicated generally by 342, such that the user may select, using a provided interface, either automatic sizing by the knee replacement application 40 or one of various available sizes of femoral implants. Thus, at step 168, in response to receiving a desired femoral implant size, the knee replacement application 40 automatically updates the displayed femoral resection surfaces of the guide on the knee image data 26 corresponding to the selected implant size.

At decisional step 170, a determination is made whether a distal shift of the femoral implant is desired. If a distal shift of the femoral implant is not desired, the method proceeds from step 70 to decisional step 176. If a distal shift of the femoral implant is desired, the method proceeds from step 170 to step 172, where the knee replacement application 40 receives a desired or requested distal shift of the femoral implant guide. For example, as best illustrated in FIGURE 9, the knee replacement application 40 provides an interface, indicated generally by 344, such as a slide bar or other type of interface, for receiving distal shift input information from the user. At step 174, the knee replacement application 40 automatically updates the femoral resection surfaces of the guide on knee image data 26 corresponding to the requested distal shift.

At decisional step 176, a determination is made whether an anterior/posterior shift of the femoral implant guide is desired. If an anterior/posterior shift of the femoral implant guide is not desired, the method proceeds from step 176 to 182. If an anterior/posterior shift of the femoral implant guide is desired, the method proceeds from step 176 to step 178, where the knee replacement application 40 receives a desired or requested anterior/posterior shift of the femoral implant guide. For example, as best illustrated in FIGURE 9, the knee replacement application 40 provides an interface, indicated generally by 346, such as a slide bar or other type of interface, for receiving anterior/posterior shift input information from the user. At step 180, the knee replacement application 40 automatically updates the femoral resection surfaces of the guide on the knee image data 26 corresponding to the requested anterior/posterior shift.

Upon completion of femoral implant sizing, the knee replacement application 40 stores the femoral implant size and location data as data 62. In FIGURE 2, the femoral implant sizing indicate a serial or sequential series of steps; however, it should be understood that each of the femoral implant sizing steps may be performed in parallel and in any order.

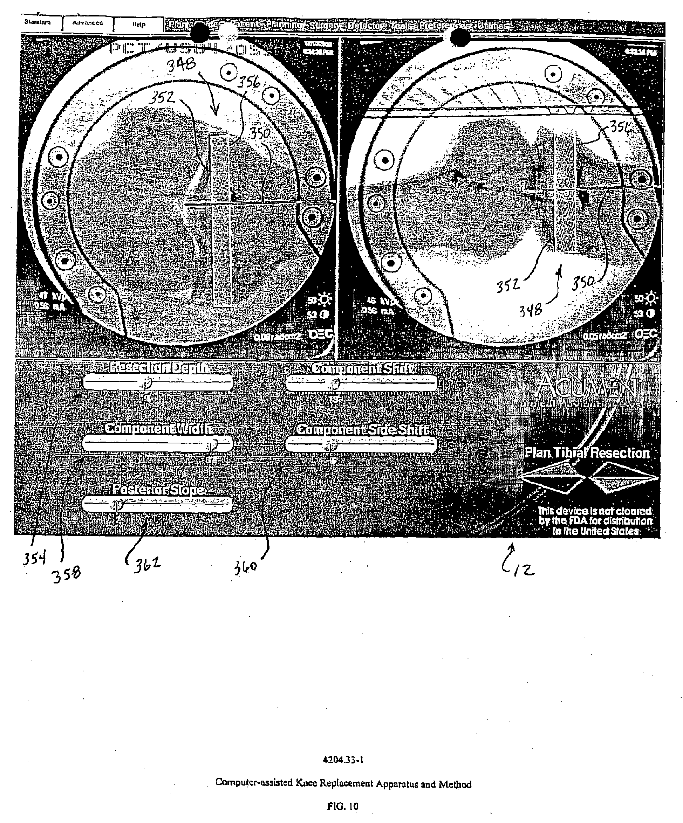

At step 184, the knee replacement application 40 displays a tibial resection planning guide 348 relative to the knee image data, as best illustrated in FIGURE 10. At step 186, the knee replacement application 40 requests and receives placement of the proximal end of the tibial resection planning guide on the tibial plateau of the subject. For example, as best illustrated in FIGURE 10, the line indicated by 350 represents the tibial mechanical axis. The left-most portion of the tibial resection planning guide, indicated by 352, represents the proximal end of the planning guide which, at step 186, is located on the tibial plateau of the subject. At step 188, the knee replacement application 40 requests and receives a desired tibial resection depth. For example, as illustrated in FIGURE 10, a slide bar or other type of interface, indicated generally by 354, may be provided for receiving a desired tibial resection depth from the user. At step 190, the knee replacement application 40 automatically updates the tibial planning guide 348 and displays the desired resection depth on the knee image data 26. For example, as illustrated in FIGURE 10, changes to the tibial resection depth are reflected by movement of the distal surface 356 of the tibial resection planning guide relative to the proximal portion 352 which remains positioned at the tibial plateau.

At step 192, the knee replacement application 40 retrieves implant data 60 corresponding to available tibial implants. For example, the tibial implant data 60 may comprise information corresponding to the geometric characteristics of available tibial implant sizes. At step 194, the knee replacement application 40 requests and receives a desired tibial implant size. For example, as illustrated in FIGURE 10, a slide bar or other type of interface 358 is provided such that the user may vary a desired size for the tibial implant. At step 196, the knee replacement application 40 automatically updates the tibial planning guide 348 and displays the desired tibial implant size on the knee image data 26. At step 198, the knee replacement application 40 requests and receives a desired tibial implant medial/lateral shift. Referring to FIGURE 10, the knee replacement application 40 provides a slide bar or other type of interface 360 such that the user may select medial or lateral shifting of the tibial implant guide relative to the knee image data 26. At step 200, the knee replacement application 40 automatically updates the tibial planning guide 348 and displays the desired tibial implant medial/lateral shift on the knee image data 26.

At step 202, the knee replacement application 40 requests and receives a desired tibial implant posterior slope. For example, as illustrated in FIGURE 10, the knee replacement application 40 provides an interface 362, such as a slide bar or other type of interface, for receiving an input from the user corresponding to a desired tibial implant posterior slope. At step 204, the knee replacement application 40 automatically updates the tibial planning guide 348 and displays the desired tibial implant posterior slope on the knee image data 26. At step 206, if sizing of the tibial implant is complete, the knee replacement application 40 stores the information corresponding to the size and location of the desired tibial implant and the tibial resection plane as tibial implant size/location data 64.

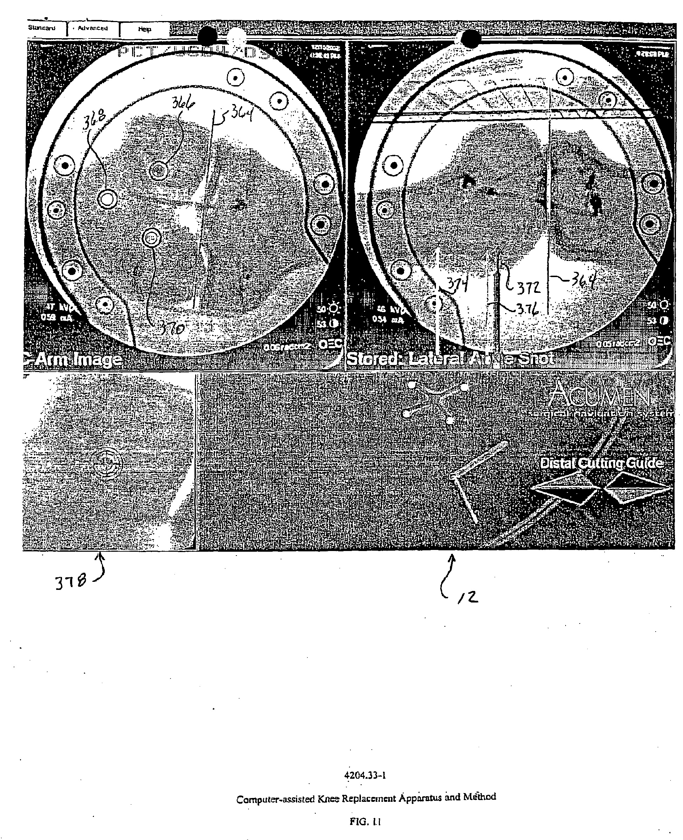

At step 208, the knee replacement application 40 retrieves femoral distal resection guide data 66. For example, the femoral distal resection guide data 66 may comprise information associated with geometric characteristics of a femoral distal resection guide such that the femoral distal resection guide may be located relative to the subject's femur corresponding to a desired distal femoral resection plane. At step 210, the knee replacement application 40 determines the resection guide pin locations and trajectories corresponding to the desired femoral resection plane. For example, based on a desired location of the femoral resection plane, the knee replacement application 40 automatically determines the locations and trajectories of the attachment pins of the resection guide for placement relative to the femur of the subject to accurately locate and guide distal femoral resection. At step 212, the knee replacement application 40 displays the distal femoral resection guide pin locations and trajectories relative to the knee image data 26, as best illustrated in FIGURE 11. As illustrated in FIGURE 11, the line indicated by 364 represents the distal femoral resection plane. The indicators 366, 368 and 370 in the anterior/posterior image view represent the pin locations for the distal resection guide. The arrows indicated by 372, 374 and 376 in the medial/lateral image view represent the pin trajectories for the distal femoral resection guide corresponding to indicators 366, 368 and 370, respectively. At step 214, the tracking system 22 acquires tracking data for a trackable tool 20, such as a drill guide, to accommodate alignment of the drill guide to the pin locations and trajectories. For example, as illustrated in the lower left hand corner of FIGURE 11 by 378, the tracking system 22 and knee replacement application 40 monitor and display, in real-time, alignment of the trackable drill guide with a selected pin location and trajectory. For example, as illustrated in the lower left hand corner of FIGURE 11 by 378, as the location of the drill guide becomes aligned with a particular pin location, the knee replacement application 40 may indicate the alignment by placing a crosshair in the alignment image. Additionally, as the orientation of the drill guide becomes aligned with a particular pin trajectory, the knee replacement application 40 may display a bullseye corresponding to the selected pin location. The knee replacement application 40 may also otherwise indicate alignment of the drill guide with a particular pin location and trajectory, such as, but not limited to, audible and/or visual signals. Thus, at step 216, the knee replacement application 40 automatically monitors and displays location and orientation of the trackable drill guide relative to the pin locations and trajectories for the resection guide. At decisional step 218, the knee replacement application 40 determines whether the drill guide is aligned with a particular pin location and trajectory. If the drill guide is not aligned with the particular pin location and trajectory, the method returns to step 216. If the drill guide is aligned with a particular pin location and trajectory, the method proceeds from step 218 to step 220, where the knee replacement application 40 signals drill guide alignment. The user may then continue to drill the pin mounting holes for the femoral resection guide and mount the femoral resection guide to the femur. The user may then continue with femoral resection. Additionally, a probe, such as a flat probe or another type of probe, may be used to verify guide alignment. For example, the probe may be disposed within a slot or otherwise placed relative to the resection guide and tracked using tracking system 22 such that positional parameters of the guide are shown on display device 12, thereby enabling the user to verify guide alignment and further align the guide as desired. Further, the probe may also be used and tracked after resection to verify resection parameters.

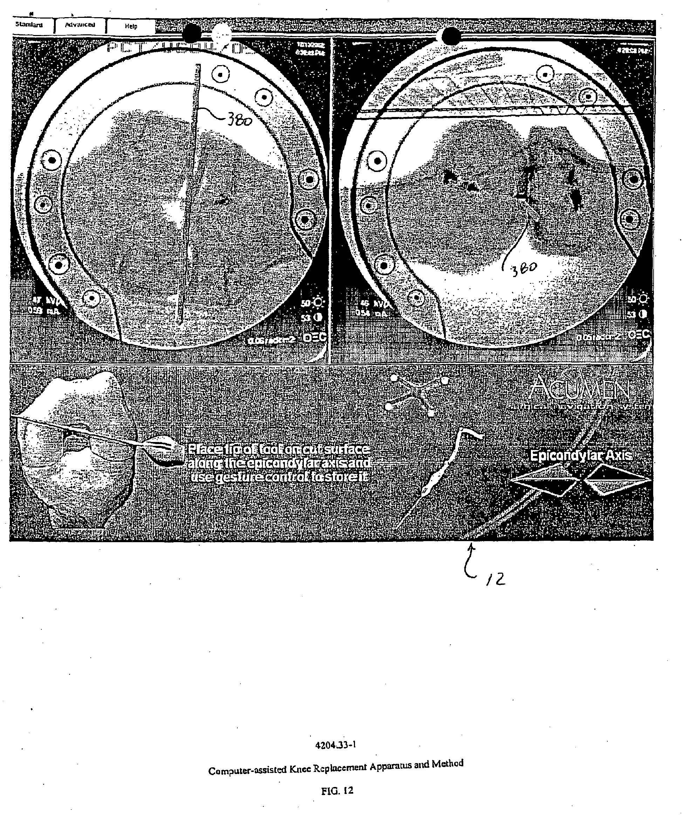

At step 222, the knee replacement application 40 requests placement of a trackable tool 20 along the epicondylar axis of the resected femur, as best illustrated in FIGURE 12. Alternatively, or additionally, application 40 may request placement of tool 20 along Whiteside's Line and/or various posterior points of the condoyles. At step 224, tracking system 22 tracks a location and/or indication of the tool 20 relative to the resected femur and displays the location and orientation of the tool 20 relative to the knee image data 26 on display device 12. At step 226, the knee replacement application 40 receives and stores the identification of the epicondylar axis by the user as epicondylar axis data 68 and displays the epicondylar axis on the knee image data 26. For example, in FIGURE 12, the epicondylar axis is illustrated by line.

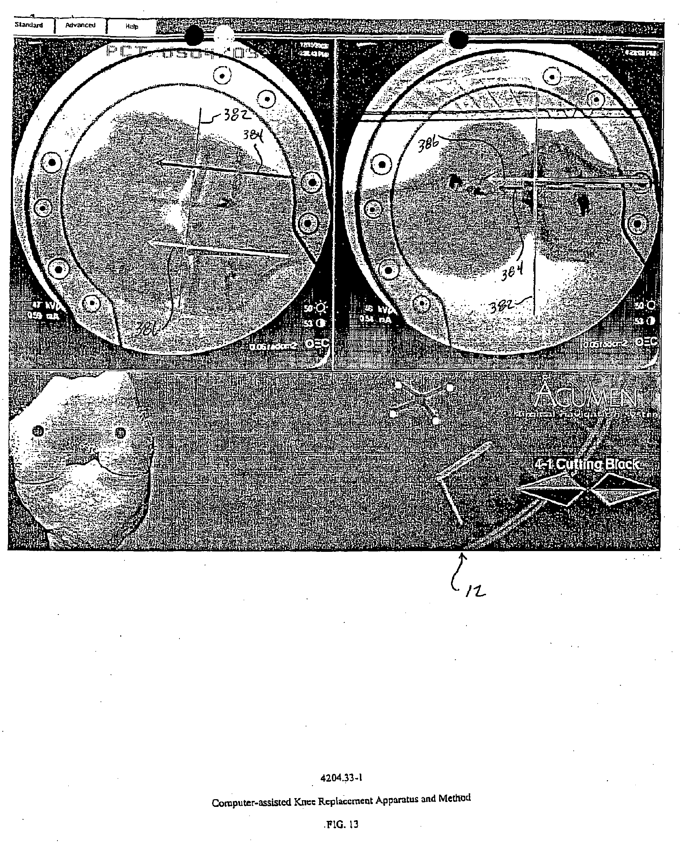

At step 228, the knee replacement application 40 retrieves femoral anterior/posterior/chamfer resection guide data 70. For example, the femoral anterior/posterior/chamfer resection guide data 70 may comprise information associated with the geometric characteristics of the resection guide such that knee replacement application 40 may locate the resection guide relative to the femur of the subject to accommodate locating the femoral anterior, posterior, and chamfer resections corresponding to a desired femoral implant. Thus, at step 230, the knee replacement application 40 determines pin trajectories for the femoral anterior/posterior/chamfer resection guide based on the epicondylar axis data 68. At step 232, the knee replacement application 40 displays the pin trajectories for the resection guide on the knee image data 26, as best illustrated in FIGURE 13. In FIGURE 13, the line indicated by 382 represents the femoral resection plane, and the arrows indicated by 384 and 386 represent the pin trajectories for the anterior/posterior/chamfer resection guide. At step 234, the tracking system 22 acquires tracking data for a trackable drill guide. At step 236, the knee replacement application 40 and tracking system 22 monitor and display alignment of the drill guide with the displayed pin trajectories for the resection guide. At decisional step 238, a determination is made whether a trackable drill guide is aligned with a particular pin trajectory for the resection guide. If the drill guide is not aligned with a pin trajectory for the resection guide, the method returns to step 236. If the drill guide is aligned with a pin trajectory for the resection guide, the method proceeds from step 238 to step 240, where the knee replacement application 40 may signal drill guide alignment by visual, audible, or other means. After alignment of the drill guide, the user may proceed with drilling the resection guide pin mounting holes and, after all mounting holes are completed, the user may mount the anterior/posterior/chamfer resection guide to the femur and perform the anterior, posterior, and chamfer resections for the femoral implant. As described above, a flat probe or other type of trackable probe may be positioned relative to the guide and/or relative to a completed resection to verify guide alignment and/or resection parameters.

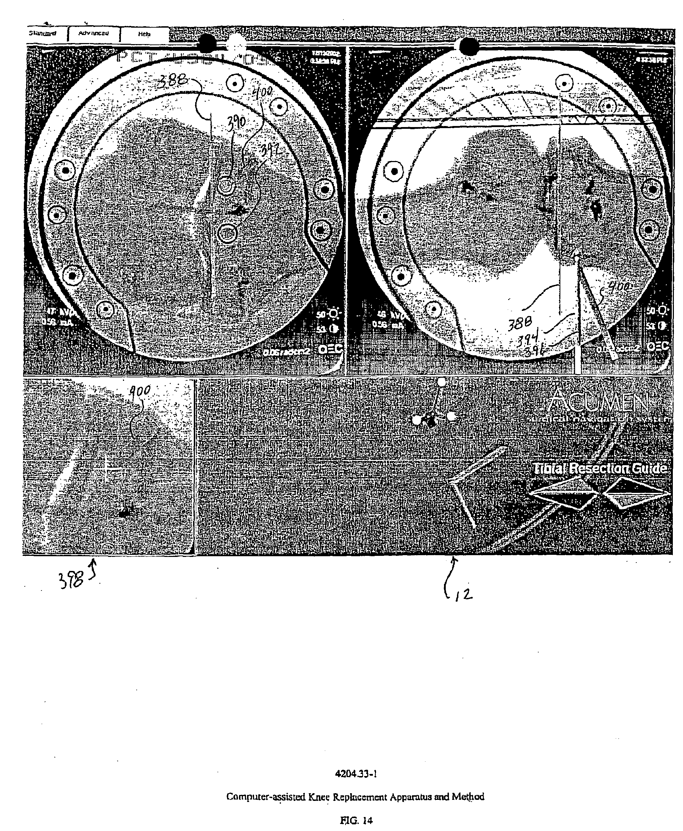

At step 242, the knee replacement application 40 retrieves tibial resection guide data 72. For example, the tibial resection guide data 72 may comprise information associated with the geometric characteristics of a tibial resection guide such that the knee replacement application 40 may locate the tibial resection guide relative to the tibia corresponding to a desired tibial resection plane. At step 246, the knee replacement application 40 determines tibial resection guide pin locations and trajectories corresponding to the desired tibial resection plane using the tibial resection guide data 72. At step 248, the knee replacement application 40 displays the tibial resection guide pin locations and trajectories on the knee image data 26, as best illustrated in FIGURE 14. In FIGURE 14, a line indicated by 388 represents the tibial resection plane, the indicators 390 and 392 represent pin locations for the tibial resection guide, and the arrows 394 and 396 represent pin trajectories for the tibial resection guide for indicators 390 and 392, respectively. As described above, a flat probe or other type of trackable probe may be positioned relative to the guide and/or relative to a completed resection to verify guide alignment and/or resection parameters.

At step 250, the tracking system 22 acquires tracking data for a drill guide. At step 252, the knee replacement application 40 and tracking system 22 monitor and display the location and orientation of the drill guide relative to the pin locations and trajectories for the tibial resection guide on the displayed knee image data 26. At decisional step 254, a determination is made whether the drill guide is aligned with a pin location and trajectory for the tibial resection guide. If the drill guide is not aligned with a pin location and trajectory for the tibial resection guide, the method returns to step 252. If the drill guide is aligned with the pin location and trajectory for the tibial resection guide, the method proceeds from step 254 to step 256, where the knee replacement application 40 signals drill guide alignment. For example, as described above, the knee replacement application 40 may provide an audible, visual, or other type of signal indicating alignment. The knee replacement application 40 may also display alignment of the drill guide with a pin location and trajectory of the tibial resection guide with a crosshair and bullseye, similar to as described above in connection with FIGURE 11, and as illustrated in the lower left hand corner of FIGURE 14 by 398. For example, in FIGURE 14, the line indicated by 400 represents the position and alignment of the drill guide relative to a particular pin location and trajectory. After alignment of the drill guide with a particular tibial resection guide pin location and trajectory, the user may drill the pin mounting hole and, after completion of all mounting holes, mount the tibial resection guide to the tibia of the subject and perform the tibial resection.

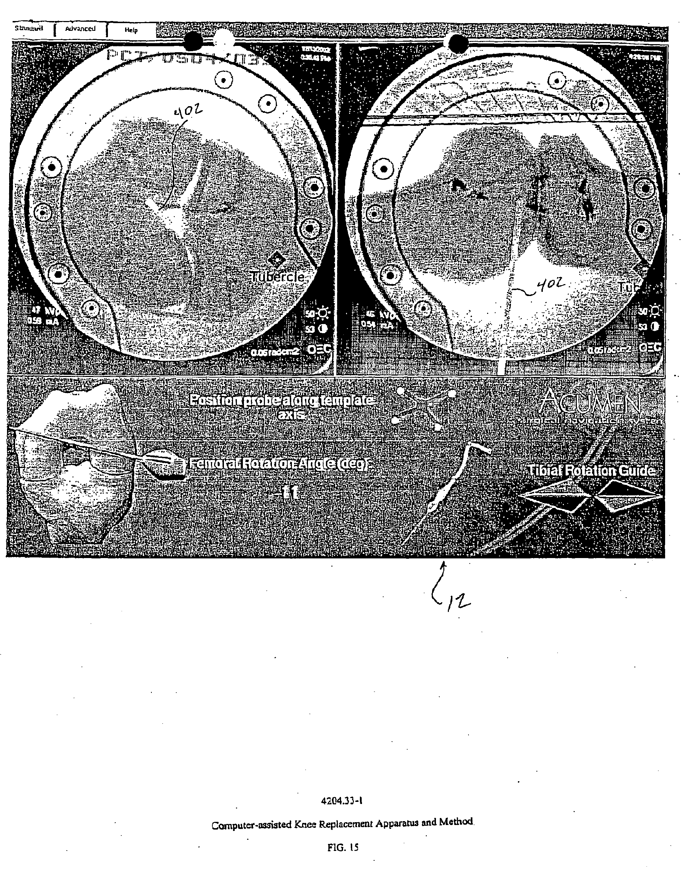

At step 258, the knee replacement application 40 requests probe placement along a tibial template axis. For example, after completion of the tibial resection, trial femoral and tibial implants may be located on the subject and the trackable probe placed at a particular orientation or position relative to one of the trial implants, indicated generally by 402, to obtain tibial and/or femoral rotation angle information. At step 260, the knee replacement application 40 in cooperation with the tracking system 22 acquires tracking data for the probe and displays the tracking data of the position and orientation of the probe relative to the knee image data 26, as best illustrated in FIGURE 15. At step 262, the knee replacement application 40 determines and displays the femoral rotation angle based on the probe angular alignment with the tibial template axis. At step 264, the knee replacement application 40 requests identification of the tibial tubercle with the trackable probe. At step 266, the tracking system 22 acquires tracking data for the probe. At step 268, the knee replacement application 40 receives and stores identification of the tibial tubercle using the probe. At step 270, the knee replacement application 40 determines and displays a tibial rotation angle based on the probe alignment with an axis formed by the tibial tubercle and its projection onto the tibial mechanical axis. The user may then adjust the femoral and/or tibial implants to a desired femoral and tibial rotation angle.

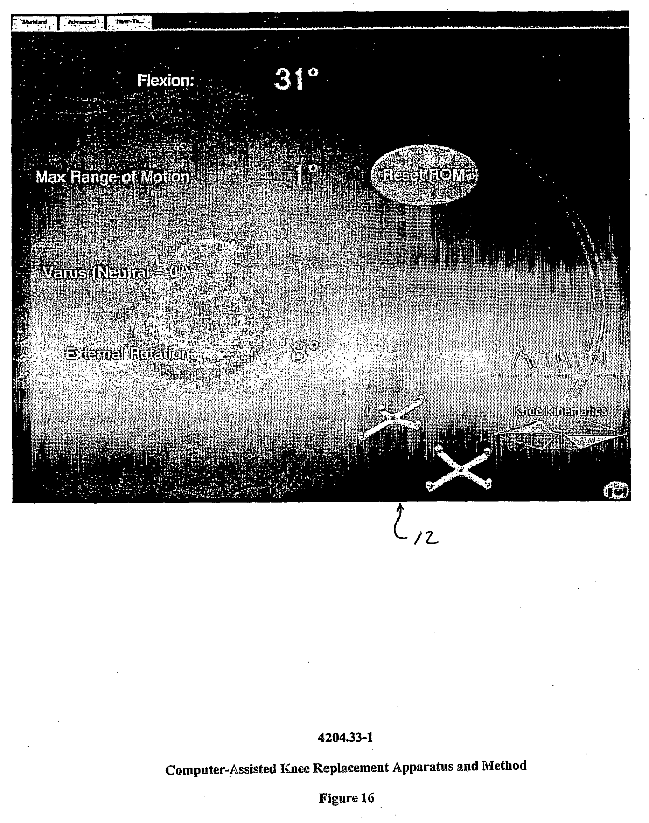

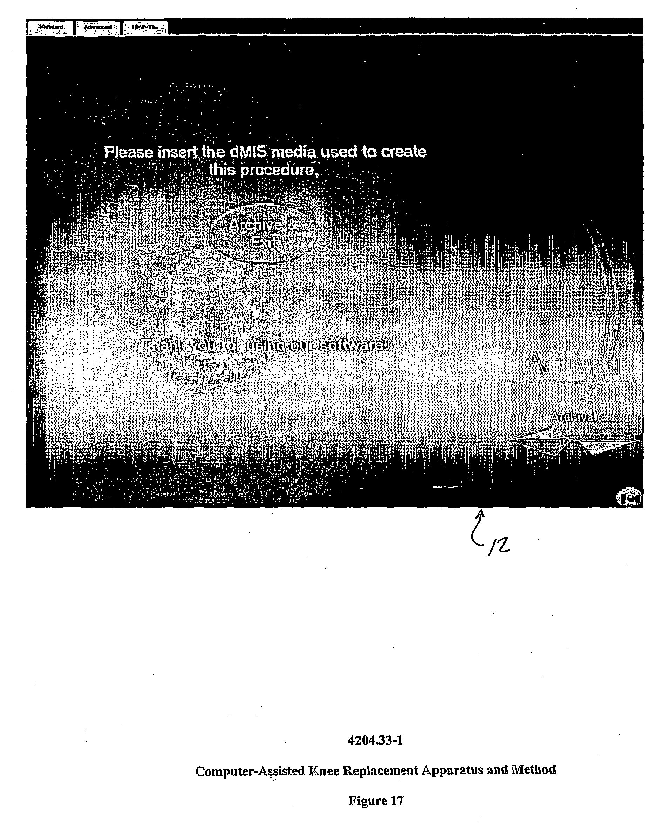

Additionally, as best illustrated in FIGURE 16, kinematics of the tibia a femur may also be monitored after resection. For example, after tibial and femur resections have been performed, flexion, varus and external rotation angles between the tibia and femur may be monitored and reported to the user, via display device 12 or otherwise, to assess soft tissue imbalance, selection of implants, and/or the overall result of the procedure. The application 40 may also be configured to prompt the user regarding archival of data generated or otherwise associated with the procedure to a disk drive or other type of storage medium, as best illustrated in FIGURE 17.

## Claims

1. A computer-assisted knee replacement apparatus, comprising:
a display device (12);
an input device (14);
a fluoroscopic imager (28) for generating images of the knee during the total knee replacement procedure;
a tracking system (22) for tracking surgical tools to provide real-time knee implant location assistance to the user during a total knee replacement procedure;
a storage medium (18) storing a knee replacement application which, when executed by a processor (16), displays on the display a series of interface images for assisting a user with the total knee replacement procedure, **characterized in that** the knee replacement application is configured to:
display a femoral implant sizing guide for performing the total knee replacement procedure;
display a listing of imaging devices for selection by a user for performing the total knee replacement procedure;
display a calibration grid for the imaging device selected by the user;
determine a calibration factor for the imaging device selected by the user;
determine a hip center, an ankle center, and a knee center for a subject for performing the total knee replacement procedure;
display a center indicator for identification of the hip center, the ankle center, and the knee center of the subject for performing the total knee replacement procedure;
receive adjustments from the user of the hip center, the ankle center, and the knee center for the subject for performing the total knee replacement procedure;
determine a femoral mechanical axis for the subject knee from the hip center and the knee center;
determine a tibial mechanical axis for the subject knee from the knee center and the ankle center;
receive an identification of a posterior condyle and an anterior cortex of a subject femur;
automatically determine a femoral resection plane corresponding to a particular femoral implant from the posterior condyle and the anterior cortex of the subject femur;
provide an interface for shifting a location of a representation of a femoral implant relative to the subject knee; and receive a requested anterior and/or distal shift of the femoral implant guide relative to the subject knee.

2. The apparatus of Claim 1, wherein the knee replacement application is adapted to receive a selection of either a right knee or a left knee from a user for performing the total knee replacement procedure.

3. The apparatus of Claim 1, wherein the knee replacement application is adapted to display three-dimensional image data of the subject for performing the total knee replacement procedure.

4. The apparatus of Claim 1, wherein the knee replacement application is adapted to determine a femoral implant size for the subject femur based on physical characteristics of the subject femur selected by the user.

5. The apparatus of Claim 1, wherein the knee replacement application is adapted to automatically update the femoral resection planes relative to the subject knee in response to a selection of a different size of femoral implant by the user.

6. The apparatus of Claim 1, wherein the knee replacement application is adapted to automatically determine pin trajectories and locations for securing a femoral resection guide relative to the subject knee corresponding to a desired femoral implant.

7. The apparatus of Claim 6, wherein the knee replacement application is adapted to cooperate with the tracking system to display real-time alignment information of a drill guide relative to the pin trajectories and locations.

8. The apparatus of Claim 1, wherein the knee replacement application is configured to:
display a tibial resection planning guide relative to the subject knee;
display an interface to the user for variably selecting a tibial implant size for the subject knee;
receive a desired tibial implant size from the user for performing the total knee replacement procedure;
receive a desired tibial resection depth from the user for performing the total knee replacement procedure;
automatically update a displayed tibial resection planning guide in response to receiving a selection of the desired tibial resection depth;
cooperate with the tracking system to display real-time alignment information for a tibial resection guide relative to the subject knee corresponding to a particular tibial implant.

9. The apparatus of Claim 8, wherein the knee replacement application is further configured to:
automatically determine pin trajectories and locations for securing a tibial resection guide relative to the subject knee corresponding to a desired tibial implant.

10. The apparatus of Claim 1, further including:
a haptic interface for feeling back information to a surgeon whether he is nearing an object or is on course.

11. The apparatus of Claim 1, wherein the knee replacement application recognizes an instrument picked up by a surgeon and moves to a part of the knee replacement application in which the picked up tool is used.

## Patentansprüche

1. Computerunterstützte Knieersatzvorrichtung, die aufweist:
eine Anzeigevorrichtung (12);
eine Eingabevorrichtung (14);
eine Röntgenabbildungseinrichtung (28) für das Erzeugen von Bildern vom Knie während des Verfahrens zum totalen Knieersatz;
ein Ortungssystem (22) für das Orten von chirurgischen Werkzeugen, um dem Benutzer eine Echtzeitknieimplantatortungsunterstützung während des Verfahrens zum totalen Knieersatz zu gewähren;
ein Speichermedium (18), das eine Knieersatzapplikation speichert, die, wenn sie von einem Prozessor (16) ausgeführt wird, auf der Anzeige eine Reihe von Schnittstellenbildern zur Unterstützung eines Benutzers bei dem Verfahren zum totalen Knieersatz anzeigt, **dadurch gekennzeichnet, dass** die Knieersatzapplikation ausgebildet ist, um:
eine Oberschenkelimplantatgrößenführung für das Durchführen des Verfahrens zum totalen Knieersatz anzuzeigen;
eine Auflistung von Abbildungsvorrichtungen für eine Auswahl durch einen Benutzer für das Durchführen des Verfahrens zum totalen Knieersatz anzuzeigen;
ein Eichgitter für die vom Benutzer ausgewählte Abbildungsvorrichtung anzuzeigen;
einen Eichfaktor für die vom Benutzer ausgewählte Abbildungsvorrichtung zu ermitteln;
eine Hüftmitte, eine Knöchelmitte und eine Kniemitte für einen Patienten für das Durchführen des Verfahrens zum totalen Knieersatz zu ermitteln;
einen Mittenindikator für die Identifizierung der Hüftmitte, der Knöchelmitte und der Kniemitte des Patienten für das Durchführen des Verfahrens zum totalen Knieersatz anzuzeigen;
Einstellungen vom Benutzer betreffs der Hüftmitte, der Knöchelmitte und der Kniemitte für den Patienten für das Durchführen des Verfahrens zum totalen Knieersatz zu erhalten;
eine mechanische Achse des Oberschenkels für das Knie des Patienten von der Hüftmitte und der Kniemitte zu ermitteln;
eine mechanische Achse des Schienbeines für das Knie des Patienten von der Kniemitte und der Knöchelmitte zu ermitteln;
eine Identifizierung des posterioren Gelenkhöckers und eines anterioren Kortex eines Oberschenkels des Patienten zu erhalten;
eine Oberschenkelresektionsebene entsprechend einem speziellen Oberschenkelimplantat vom posterioren Gelenkhöcker und anterioren Kortex des Oberschenkels des Patienten automatisch zu ermitteln;
eine Schnittstelle für das Verschieben einer Position einer Darstellung eines Oberschenkelimplantates relativ zum Knie des Patienten zu liefern; und
eine angeforderte anteriore und/oder distale Verschiebung der Oberschenkelimplantatführung relativ zum Knie des Patienten zu erhalten.

2. Vorrichtung nach Anspruch 1, bei der die Knieersatzapplikation angepasst ist, um eine Auswahl von entweder einem rechten Knie oder einem linken Knie von einem Benutzer für das Durchführen des Verfahrens zum totalen Knieersatz zu erhalten.

3. Vorrichtung nach Anspruch 1, bei der die Knieersatzapplikation angepasst ist, um dreidimensionale Bilddaten des Patienten für das Durchführen des Verfahrens zum totalen Knieersatz anzuzeigen.

4. Vorrichtung nach Anspruch 1, bei der die Knieersatzapplikation angepasst ist, um eine Oberschenkelimplantatgröße für den Oberschenkel des Patienten auf der Grundlage von des vom Benutzer ausgewählten körperlichen Eigenschaften Oberschenkels des Patienten zu ermitteln.

5. Vorrichtung nach Anspruch 1, bei der die Knieersatzapplikation angepasst ist, um die Oberschenkelresektionsebenen relativ zum Knie des Patienten als Reaktion auf eine Auswahl einer unterschiedlichen Größe des Oberschenkelimplantates durch den Benutzer automatisch zu aktualisieren.

6. Vorrichtung nach Anspruch 1, bei der die Knieersatzapplikation angepasst ist, um Pin-Verlaufsbahnen und -Positionen für das Sichern einer Oberschenkelresektionsführung relativ zum Knie des Patienten entsprechend einem gewünschten Oberschenkelimplantat automatisch zu ermitteln.

7. Vorrichtung nach Anspruch 6, bei der die Knieersatzapplikation angepasst ist, um mit dem Ortungssystem zusammenzuwirken, um eine Echtzeitausrichtungsinformation einer Bohrerführung relativ zu den Pin-Verlaufsbahnen und -Positionen anzuzeigen.

8. Vorrichtung nach Anspruch 1, bei der die Knieersatzapplikation ausgebildet ist, um:
eine Schienbeinresektionsplanungsführung relativ zum Knie des Patienten anzuzeigen;
eine Schnittstelle für den Benutzer für das veränderliche Auswählen einer Schienbeinimplantatgröße für das Knie des Patienten anzuzeigen;
eine gewünschte Schienbeinimplantatgröße vom Benutzer für das Durchführen des Verfahrens zum totalen Knieersatz zu erhalten;
eine gewünschte Schienbeinresektionstiefe vom Benutzer für das Durchführen des Verfahrens zum totalen Knieersatz zu erhalten;
eine angezeigte Schienbeinresektionsplanungsführung als Reaktion auf das Erhalten einer Auswahl der gewünschten Schienbeinresektionstiefe automatisch zu aktualisieren;
mit dem Ortungssystem zusammenzuarbeiten, um eine Echtzeitausrichtungsinformation für eine Schienbeinresektionsführung relativ zum Knie des Patienten entsprechend einem speziellen Schienbeinimplantat anzuzeigen.

9. Vorrichtung nach Anspruch 8, bei der die Knieersatzapplikation außerdem ausgebildet ist, um:
die Pinverlaufsbahnen und -Positionen für das Sichern einer Schienbeinresektionsführung relativ zum Knie des Patienten entsprechend einem gewünschten Schienbeinimplantat automatisch zu ermitteln.

10. Vorrichtung nach Anspruch 1, die außerdem umfasst:
eine haptische Schnittstelle für das Rückführen einer Information zu einem Chirurgen, ob er sich einem Objekt nähert oder auf Kurs ist.

11. Vorrichtung nach Anspruch 1, bei der die Knieersatzapplikation ein von einem Chirurgen aufgenommenes Instrument erkennt und zu einem Abschnitt der Knieersatzapplikation bewegt, in dem das aufgenommene Werkzeug benutzt wird.

## Revendications

1. Appareil de remplacement d'un genou assisté par ordinateur, comprenant:
un dispositif d'affichage (12);
un dispositif d'entrée (14);
un dispositif imageur fluoroscopique (28) pour produire des images du genou au cours de la procédure de remplacement total du genou ;
un système de suivi (22) pour assurer le suivi des outils chirurgicaux, pour fournir une assistance en temps réel concernant l'emplacement de l'implant du genou à l'utilisateur au cours de la procédure de remplacement total du genou ;
un support d'enregistrement (18) pour enregistrer une application de remplacement du genou, qui, lors de son exécution par un processeur (16), affiche sur l'affichage une série d'images d'interface pour assister un utilisateur lors de la procédure de remplacement total du genou, **caractérisé en ce que** l'application de remplacement du genou est configurée de sorte à :
afficher un guide de dimensionnement de l'implant fémoral en vue de l'exécution de la procédure de remplacement total du genou;
afficher une liste de dispositifs imageurs en vue d'une sélection par un utilisateur, afin d'exécuter la procédure de remplacement total du genou ;
afficher une grille de calibrage pour le dispositif imageur sélectionné par l'utilisateur ;
déterminer un facteur de calibrage pour le dispositif imageur sélectionné par l'utilisateur ;
déterminer un centre de la hanche, un centre de la cheville et un centre du genou pour un sujet en vue de l'exécution de la procédure de remplacement total du genou ;
afficher un indicateur des centres pour identifier le centre de la hanche, le centre de la cheville et le centre du genou du sujet en vue de l'exécution de la procédure de remplacement total du genou ;
recevoir des ajustements de la part de l'utilisateur concernant le centre de la hanche, le centre de la cheville et le centre du genou pour le sujet, en vue de l'exécution de la procédure de remplacement total du genou ;
déterminer un axe fémoral mécanique pour le genou du sujet sur la base du centre de la hanche et du centre du genou ;
déterminer un axe tibial mécanique pour le genou du sujet sur la base du centre du genou et du centre de la cheville ;
recevoir une identification du condyle postérieur et du cortex antérieur d'un fémur du sujet ;
déterminer automatiquement un plan de résection fémorale correspondant à un implant fémoral particulier sur la base du condycle postérieur et du cortex antérieur du fémur du sujet ;
fournir une interface pour déplacer un emplacement d'une représentation d'un implant fémoral par rapport au genou du sujet ; et
recevoir un déplacement antérieur et/ou distal requis du guide de l'implant fémoral par rapport au genou du sujet.

2. Appareil selon la revendication 1, dans lequel l'application de remplacement du genou est adaptée pour recevoir une sélection d'un genou de droite ou d'un genou de gauche d'un utilisateur en vue de l'exécution de la procédure de remplacement total du genou.

3. Appareil selon la revendication 1, dans lequel l'application de remplacement du genou est adaptée pour afficher des données d'image tridimensionnelles du sujet en vue de l'exécution de la procédure de remplacement total du genou.

4. Appareil selon la revendication 1, dans lequel l'application de remplacement du genou est adaptée pour déterminer une taille d'un implant fémoral pour le fémur du sujet, sur la base de caractéristiques physiques du fémur du sujet sélectionnées par l'utilisateur.

5. Appareil selon la revendication 1, dans lequel l'application de remplacement du genou est adaptée pour mettre à jour automatiquement les plans de résection fémorale par rapport au genou du sujet en réponse à une sélection d'une taille différente de l'implant fémoral par l'utilisateur.

6. Appareil selon la revendication 1, dans lequel l'application de remplacement du genou est adaptée pour déterminer automatiquement des trajectoires et des emplacements de broches pour fixer un guide de résection fémorale par rapport au genou du sujet, correspondant à un implant fémoral voulu.

7. Apapreil selon la revendication 6, dans lequel l'application de remplacement du genou est adaptée pour coopérer avec le système de suivi pour afficher des informations d'alignement en temps réel d'un guide de perçage par rapport aux trajectoires et aux emplacements des broches.

8. Appareil selon la revendication 1, dans lequel l'application de remplacement du genou est configurée de sorte à :
afficher un guide de planification de la résection tibiale par rapport au genou du sujet ;
afficher une interface à l'intention de l'utilisateur, pour sélectionner de manière variable une taille de l'implant tibial pour le genou du sujet ;
recevoir une taille de l'implant tibial voulu de la part de l'utilisateur en vue de l'exécution de la procédure de remplacement total du genou ;
recevoir une profondeur de la résection tibiale voulue de la part de l'utilisateur en vue de l'exécution de la procédure de remplacement total du genou ;
mettre à jour automatiquement un guide de planification de la résection tibiale affiché en réponse à la réception d'une sélection de la profondeur de résection tibiale voulue ;
coopérer avec le système de suivi pour afficher des informations d'alignement en temps réel pour un guide de résection tibiale par rapport au genou du sujet, correspondant à un implant tibial particulier.

9. Appareil selon la revendication 8, dans lequel l'application de remplacement du genou est en outre configurée de sorte à :
déterminer automatiquement les trajectoires et les emplacements de broches pour fixer un guide de résection tibiale par rapport à un genou du sujet, correspondant à un implant tibial voulu.

10. Appareil selon la revendication 1, englobant en outre :
une interface haptique pour fournir des informations de retour à un chirurgien, pour l'informer du fait qu'il se rapproche d'un objet ou s'il s'oriente vers celui-ci.

11. Appareil selon la revendication 1, dans lequel l'application de remplacement du genou reconnaît un instrument prélevé par un chirurgien et se déplace vers une partie de l'application de remplacement du genou dans laquelle l'outil prélevé est utilisé.
